# EUROPEAN PATENT APPLICATION

(11) **EP 1 082 947 A1**
(43) Date of publication of application: **14.03.2001**
(21) Application number: 99890290.2
(22) Date of filing: 09.09.1999
(51) Int. Cl.: A61F 2/06

(54) **Intraluminal graft with intraluminal access**

(71) Applicant: Grampp, Stephan, Dr. med., 1180 Wien (AT); Henk, Christine, Dr. med., 1180 Wien (AT)
(72) Inventor: Grampp, Stephan, Dr.med., 1180 Wien (AT); Henk, Christine, Dr.med., 1180 Wien (AT); Lammer, Johannes, Prof.Dr.med., 1230 Wien (AT)

(57) **Abstract**

An intraluminal graft (stent)(1) is placed into the body by means of a catheter or introduction set. Agents can be installed in the interior lumen of the graft through a punctured membrane (3) fixed to the inner surface of the graft. The inner membrane of the graft can be accessed through a port (2) on the outer surface of said graft. By applying a volume of an agent of any sorts (gas,liquid) into said port, the agent will penetrate through the perforated inner membrane. This can on the one hand be helpful if the installed agent is a drug or medication one wishes to install at the desired lumen. On the other hand it may be an agent, which penetrates through the wall of said graft and may aid in cleaning of the inner aspect of the graft by loosening clotted residual fluids or other adhesive materials.

## Description

### BACKGROUND - FIELD OF THE INVENTION

Grafts for angioplasty of blood vessels or for use in other passageways such as bile ducts or the gastrointestinal tract have largely proved useful in medical practice for the prevention of occlusions or re-stenosis after therapy. Known grafts do for example consist of cartridge-shaped lattice of steel or plastic, which are self-expanding or balloon-expandable. The invention relates to an intraluminal graft for use within an intracorporal passageway or duct and a method for installing agents to the inside of said graft by manipulation from outside.

### BACKGROUND - DESCRIPTION OF PRIOR ART

This discovery concerns a graft, stent, or endoprosthesis, which will further be referred to solely under the term 'graft' and an apparatus for percutaneous manipulation of the graft as per the general description given in the description of the invention.

There are different kinds of stents or endoprostheses commonly called grafts, which have the common characteristic of being presented into a blood vessel, or other body cavity, or lumen via endoscopic or fluoroscopic controlled manipulation, usually by means of a guide wire. Grafts can for example consist of a cartridge-shaped lattice made of steel or of a wire mesh, or of other materials and may be porous or may be covered with an isolating, impenetrable material.

Grafts of various kinds and sizes have largely proved useful in medical practice for the therapy or prevention of occlusions or re-stenosis after transluminal placement in the blood vessels, the biliary tract, the airways, the gastrointestinal tract, or the urogenital system. Occlusion of body passages or ducts may occur by clotting of the fluids or substances transported to or through those, by tissue growth due to malignant or benign conditions, by development of scar tissue, or by compression from outside.

Intraluminal grafting has been demonstrated to present an alternative to conventional surgical approaches. It was most successfully applied in intravascular grafting, which involves the percutaneous insertion of a graft into a blood vessel and its delivery by means of a catheter to its desired location. In many cases the graft may be expanded to widen the lumen of a body passageway as for example described in U.S, Pat. 4,733,665. Other body passageways may be accessed through natural openings on the body surface or through artificial percutaneous access. In most of the approaches, grafts were expanded through use of a catheter mounted balloon. In these procedures the balloon is inflated within the stenosed structure or within a newly drilled or cut passageway through tissue.

Structures which have been previously used as intraluminal grafts have included coiled steel springs, helically wound coil springs, or expanding steel grafts in a zig-zag pattern. The inner as well as outer diameters of those mostly cylindrical structures were defined either by manufactured design specifications or by applying pressure to their inner surface upon installation and expanding the graft to a desired size.

In general, the foregoing structures have one major disadvantage in common. These structures must be delivered to the desired location in a collapsed state and then expanded to their final size and shape by either a spring mechanism, mechanical pressure via a balloon catheter or through built-in elastic memory by changing their temperature. When finally placed, those implants will in a lot of cases be overgrown by cells of the neighboring vessels or ducts and may obstruct again. A frequently encountered complication after application of a graft is that fluids or materials, which are being transported through said graft begin to clot or fall out inside the graft, adhere at the internal surface of the graft and partially or totally obstruct the lumen. Thus, conventional grafts do not necessarily provide an acceptable long-term solution in the face of progredient disease. In those cases, only a total change of the graft followed by a re-implantation of a new graft, overgrafting with a smaller or similar sized graft, or cleaning by means of catheter or balloon could be performed until now. Access to the inner surface of a graft, however, may allow for viable therapeutic method to either clean the inner surface of the graft by hydrostatic pressure or install an agent, which may support the cleaning process.

Prior to the development of the present invention, there has been no intraluminal graft and apparatus which allows for an intraluminal access through the graft. Therefore, the art has sought an intraluminal graft and method for installing agents inside the graft. These may aid for example in maintaining the passage through the graft or also may be transported, together with passing materials (i.e. blood), to a location in the host where their presence is desired.

### SUMMARY OF THE INVENTION

This invention relates to devices known as grafts which provide support to a body passageway, which can be a natural passageway such as a blood vessel, or can also be an artificial passageway following a therapeutic intervention. It relates further to a method and a system for installing agents inside an intraluminal graft.

The present invention is summarized in that a specific designed graft is placed into a body by means of a catheter, introduction set, or surgical manipulation. The graft is for example a hollow, cylindrical (tube-like) structure made of a synthetic substance or can be also a wire mesh tube covered with a synthetic substance, or a wire mesh. The graft may for example be self-expanding as for example described in U.S, Pat. 5,061,275 or be balloon-expandable as for example described in U.S, Pat. 4,733,665. The structure provides stability for maintaining the size of a body passageway, thereby preventing constriction and maintaining the lumen open. The device may for example be transported and introduced into the target area by means of a catheter, a guide wire, or an endoscope. A balloon-like, ring-shaped or cushion-shaped structure fixed to the inner diameter of the graft can be accessed through a port on the outer surface of said graft. The present invention includes a tubular-shaped graft with an inner portion. An port is attached to the outer surface of the tubular-shaped graft to enable access to the inner portion from outside. Injection of agents into the inner structure can be performed by percutaneous manipulation with specially designed tools. For temporary use, such a tool may consist of a percutaneous punction needle or specifically designed access tool which can be linked to a port at the outer surface of said graft. For long-term use, such a tool may consist of a subcutaneous port, which may be permanently linked to a port at the outer surface of said graft. By applying a volume of gas, liquid, or any other suitable agent into said port, the perforated membrane attached to the inner diameter of the graft can be accessed. Injected agents are then, by means of pressure, transported through the perforated openings of the inner membrane into the lumen of the graft. The fact of the passage through the perforation may support the loosening of potential adhesive substances at the wall of the graft. Injected agents can for example further be chosen so that they have their function in bringing adhesive materials into solution. An other possible application of the invention may be the targeted installation of drugs such as chemotherapeutic agents or antibiotics, which can reach their target tissue volume in a selective manner.

A method of the present invention comprises the steps of: inserting an intraluminal graft, which may be for example disposed upon a catheter, into a body passageway into a desired location. A feature of the present invention is that agents can be installed inside the graft by external manipulation.

It is another object of the present invention to provide a intraluminal graft of the character above which can be readily and economically manufactured.

Other objects, advantages, and features of the present invention will become apparent from the following specification when taken in conjunction with the accompanying drawings. When the invention will be described in connection with the preferred embodiment, it will be understood that it is not intended to limit the invention to that embodiment. On the contrary, it is intended to cover all alternatives, modifications, and equivalents as may be included within the spirit and scope of the invention as defined by the appended claims.

### BRIEF DESCRIPTION OF THE DRAWINGS

FIG. 1. A perspective view of a graft for a body passageway with a perforated inner surface.

FIG. 2. An axial cross-sectional view of a graft for a body passageway with a perforated inner surface.

FIG. 3. A longitudinal cross-sectional view of a graft with a perforated inner surface before injection of an agent.

FIG. 4. A longitudinal cross-sectional view of the port of a graft with a perforated inner surface.

FIG. 5 a. A longitudinal cross-sectional view of a graft with a perforated inner surface after injection of an agent, pictured with an access tool (apparatus for inflation) attached.

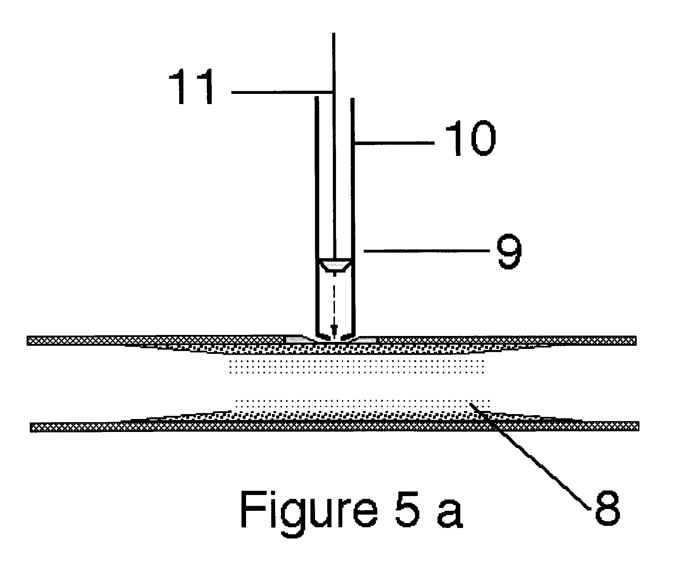

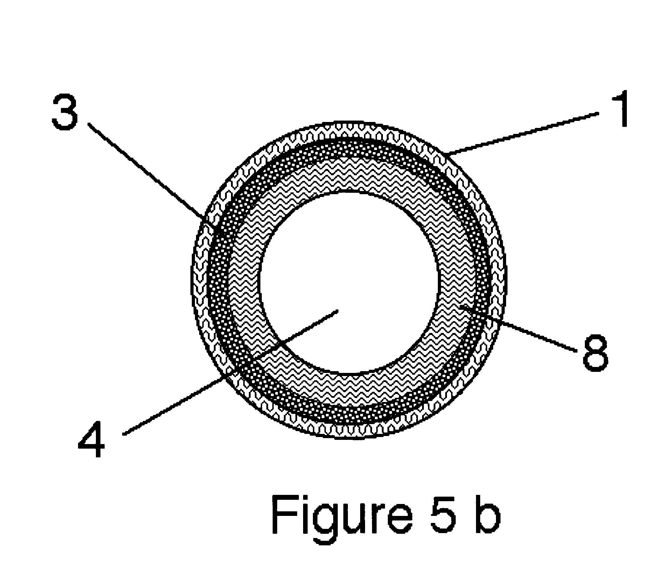

FIG. 5 b. An axial cross-sectional view of a graft with a perforated inner surface after injection of an agent, pictured with an access tool (apparatus for inflation) attached.

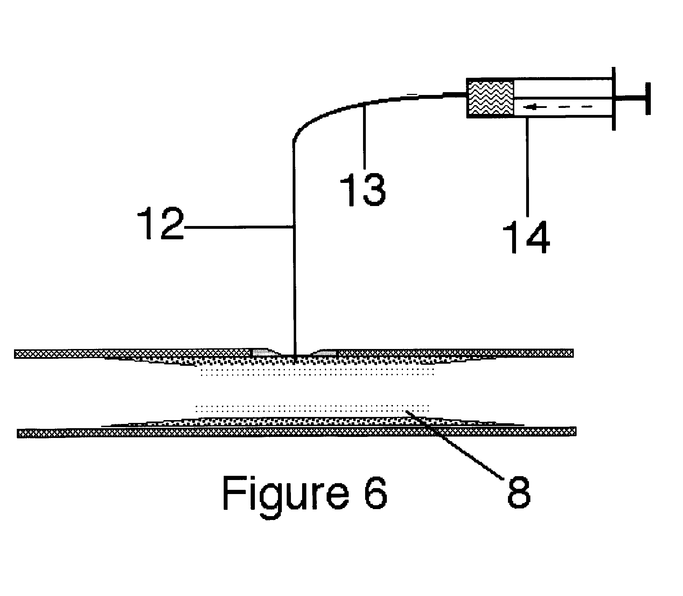

FIG. 6. A longitudinal cross-sectional view of a graft with a perforated inner surface after injection of an agent, pictured with an injection needle attached.

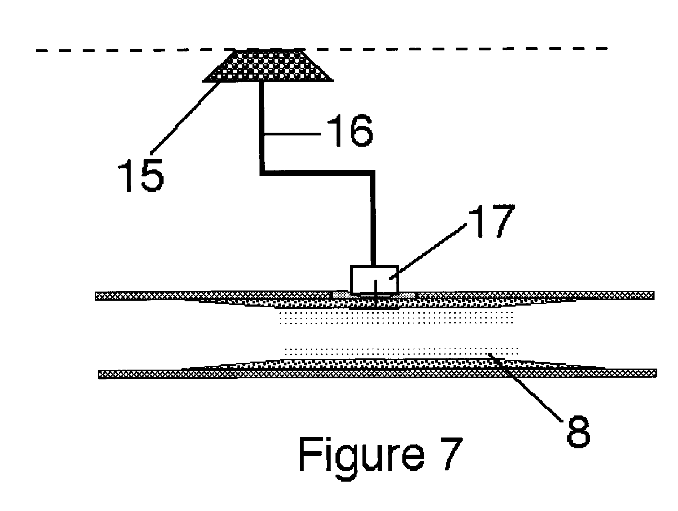

FIG. 7. A longitudinal cross-sectional view of a graft with a perforated inner surface after injection of an agent, with a subcutaneous port system attached.

### DETAILED DESCRIPTION OF THE INVENTION

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further applications of the principles of the invention as illustrated therein being contemplated as would normally occur to one skilled in the art to which the invention relates.

The invention is directed in particular towards singular or repeated installation of agents to the inside of a graft. It is further directed towards penetration of the inner graft surface by said agents to facilitate or cause the detachment of clotted substances, which may contribute to the obstruction of the inner lumen.

The use of the words prosthesis, endoprosthesis, graft, and stent encompasses the usages within various types of body passageways.

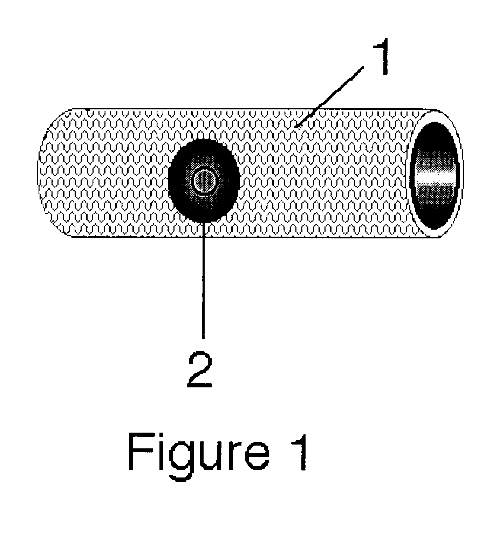

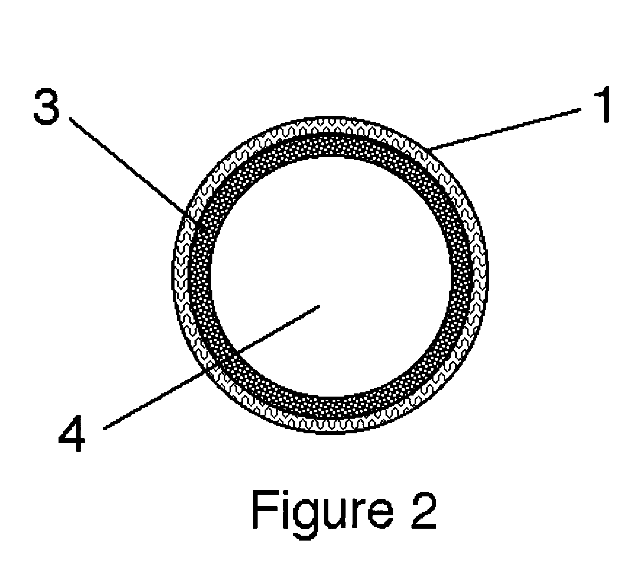

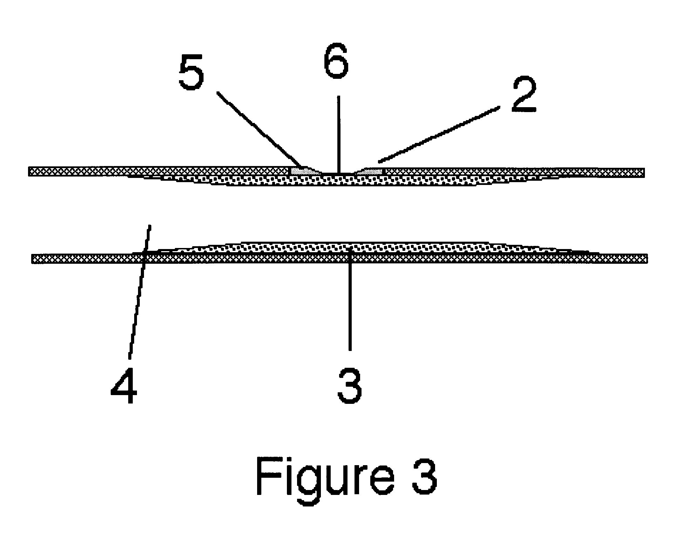

Shown in figures 1 and 2 is a graft, which implements the present invention consisting of a cuff or outer hull 1 made for example of a wire mesh, which may or may not be covered by a surface coating of material, which may have anti-thrombogenic properties (such as carbon fiber). Many of the details of how such outer cuffs are designed and produced are described in patent applications. Specifications are hereby incorporated by reference. The cuff 1 has one or more ports 2 penetrating its surface as shown in figure 3. This port 2 penetrates the cuff 1 and is used as the link between tools for outer manipulation and the perforated inner membrane 3, which is attached to the inside of the cuff 1. The perforated inner membrane 3 accordingly represents the outer boundary of the lumen 4 maintained by the graft. The perforated inner membrane 3 is attached to the outer cuff by stitching, gluing, or any other method sufficient to ensure bonding. If the cuff 1 were made of plastic, the perforated inner membrane could also be attached to the cuff by embedding in the plastic; it being understood that the means for attaching the cuffs to the perforated inner membrane can be varied depending on the cuff material and other factors without diverging from the intended scoop of the present invention.

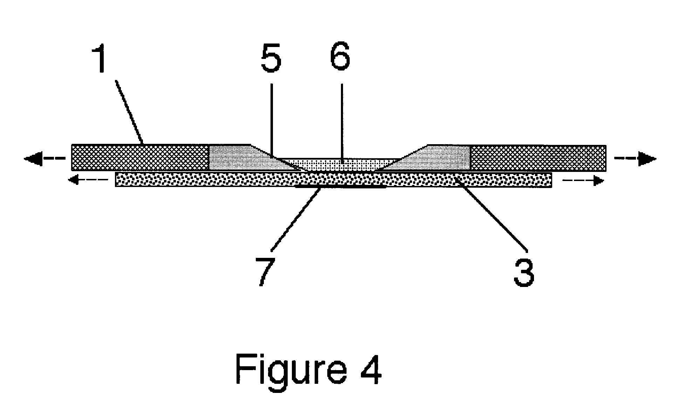

As demonstrated in figure 4, the port 2 may consist of a funnel-shaped ring 5, for example made of metal or plastic, which is embedded into or attached onto the cuff 1. The opening inside the ring 5 is covered by a self-sealing member 6, which may be for example an elastic rubber member, which is attached to the inner aspect of the ring 5. Such a member is for example described in U.S, Pat. 4,840,615. Access to the perforated inner membrane 3 is possible through the port 2 by penetrating the surface member 5 of the port 2 with a injection needle or specific access tool. In cause of use of a needle or other tool with a sharp or pointed surface the inner aspect of the inner membrane 3 may potentially be in danger of being penetrated. Therefore the inner aspect of the perforated inner membrane 3 may be strengthened and/or protected by a metal or plastic insert 7 attached to its material directly below the port 2. A pointed object may now dislocate the inner aspect of the inner membrane 3 into the lumen but will not penetrate through the fabric of the inner membrane 3.

Installation of agents 8 and passage through the perforated inner membrane 3 of the graft inside the host body as demonstrated in figure 5 a and 5 b may be accomplished from outside the body by means of manipulation through a punction tool, injection-type hollow needle, or permanent connection. In this embodiment a possible punction or access tool 9 as shown in figure 5 a comprises of a tube-like catheter 10 for example made from metal, which enables the percutaneous punction through tissue. The access tool 9 may for example be used under fluoroscopic control and connects to the port 2 on the outer surface of the cuff 1. Said port 2 has for example an outer rim or ring 5 to guide the punction tool to its desired location, which usually will be in the center of the port 2. The access tool also may comprise further of an inner portion which is a guided hollow needle-like structure 11 which fits inside of the outer hull of the punction tool. The hollow needle-like structure 11 is constructed in a fashion that if placed inside the outer hull, the tip reaches an appropriate amount over the end of said outer hull to penetrate the member 6. The distance between the end of the tube 10 and the end of the inserted needle 11 may be suited to penetrate through the outer connecting member 6 of the port 2 and reach the internal cavity above the perforated inner membrane 3. Through the needle 11 of the access tool 9 or through a regular injection needle 12, connected for example by means of a tube 13 to a syringe 14 or any other device suited for creating pressure, a fluid, gas, or other agent may be injected above and through the perforated inner membrane 3. This will lead to passage of said agent through the perforations of the inner membrane 3 as shown in figure 5 a and 5 b. Access by means of an injection-type needle is depicted in figure 6.

Injection of an agent through the inner membrane 3 of the graft 1 inside the host body as demonstrated in figure 5 a and 5 b may also be accomplished by means of a permanent connection as shown in figure 7. In this embodiment, a possible subcutaneous port 14 comprises of a cushion-like structure with a surface member, which may be accessed through percutaneous puncture with an injection-needle like device. Such a subcutaneous port 14 is for example described in U.S, Pat. 4,840,615. The subcutaneous port 14 may be connected to the port 2 on the outer surface of the cuff 1 with a tube-like catheter 16. The catheter 16 may be connected to the port 2 on the outer surface of the cuff 1 by means of a connecting part 17, which docks into the cavity of the port 2 and may have an attached hollow needle or other appropriate device on its surface to penetrate the member 6. Through a regular injection needle 12, which might be connected to a syringe 14 or any other device suited for creating pressure a fluid or a gas may be injected into the subcutaneous port 15, which is connected to the inner membrane 3 with the result of injecting an agent through the inner membrane 3. Installation of such a subcutaneous port 15 may for example be performed under fluoroscopic control or by open surgical access.

In order to provide means by which the position of the graft or cuff 1, especially the position of the port 2 or ports may be determined by fluoroscopic devices, one or more radioopaque markers may be located on the graft and in the port or ports; this may be for example a radioopaque band around the connecting member 6.

In order to provide means by which the position of the graft or cuff 1, can be determined, one or more radioopaque markers may be located on the inner surface of the inner membrane 3; this may be for example a ring shaped structure which can be visualized in an axial direction by means of fluoroscopic diagnosis. It is to understood that the present invention is not to be interpreted to be limited to use under fluoroscopic control but also to use controlled by other diagnostic modalities such as ultrasound, computed tomography, or magnetic resonance imaging. The material properties of the graft 1 as well as of potential markers or injected fluids or gases which support in determining the desired location of graft 1 and/or inner port 2 may be chosen according to different imaging requirements; these may for example be materials, which are susceptible to magnetic fields as used in magnetic resonance imaging, x-rays, or ultrasonic waves.

Using our said approach the instrument is primarily capable of providing the installation of agents to the internal surface of a graft by minimal invasive procedures.

It is to understood that the present invention is not limited to the exact details of construction, operation, exact materials or embodiment shown and described, as obvious modifications and equivalents will be apparent to one skilled in the art. Instead it encompasses all variations thereon which come within the scope of the following claims.

### REFERENCES CITED

| U.S. PATENT DOCUMENTS | | |
|---|---|---|
| 4,733,665 | 3/1988 Palmaz | 128/343 |
| 4,832,690 | 5/1989 Kuu | 604/85 |
| 4,840,615 | 6/1989 Hancock | 604/93 |
| 5,061,275 | 10/1991Walsten | 623/1 |
| 5,147,385 | 9/1992 Beck | 623/1 |

| EUROPEAN PATENT DOCUMENTS | | |
|---|---|---|
| 93250022.6 2/1987 | European Pat. Off. | A 61F 2/06 |
| 88309274.4 10/1988 | European Pat. Off. | A 61F 2/06 |
| 90101509.9 1/1990 | European Pat. Off. | A 61F 2/06 |
| 91309197.1 10/1991 | European Pat. Off. | A 61F 2/06 |
| 92305455.5 6/1992 | European Pat. Off. | A 61F 2/06 |

### OTHER PUBLICATIONS

Becker,C.D. et al. "Percutaneous palliation of malignant obstructive jaundice with the Wallstent endoprosthesis." *JVIR* 4: 597 (1993)
Culp,W.C. et al. "Biliary strictures in liver transplant receptionist: treatment with metal stents." *Radiology* 199: 339 (1996)
Jackson,J.E. et al. "Biliary endoprosthesis dysfunction in patients with malignant hilar tumors: successful treatment by percutaneous replacement of the stent." *Am J Roentgenol* 155(2): 391 (1990)
Hausegger,K.A. et al. "Benign biliary obstruction : is treatment with the Wallstent advisable ?" *Radiology* 200: 437 (1996)
Kishi,K. et al. "Dacron-covered stent therapy for portal vein tumor thrombus in hepatocellular carcinoma." *Acta Radiol* 34: 266 (1993)
Kubota,Y. et al. "Bilateral internal biliary drainage of hilar cholangiocarcinoma with modified Gianturco Z stents inserted via a single percutaneous tract" *JVIR*, 4: 605 (1993)
Palmaz,J.C. et al. "Intravascular stents: tissue-stent interactions and design considerations. " *Am J Roentgenol* , 160: 613 (1993)
Peltzer,M.Y. et al. "Treatment of Budd-Chiari syndrome with a transjugular intrahepatic portosystemic shunt." *JVIR*, 4: 263 (1993)
Rossi,P. et al. "Metallic stents in malignant biliary obstruction: results of a multicenter European study of 240 patients." JVIR, 5: 279 (1994)
Rousseau,H. et al. "Transjugular intrahepatic portosystemic shunts using the Wallstent prosthesis: follow-up study." *Cardiovasc Intervent radiol,* 17: 7 (1994)
Sapoval,M.R. et al. "Outcome of percutaneous intervention in iliac artery stents" *Radiology* 198: 481 (1996)
Vorwerk,D. et al. "Primary stent placement for chronic iliac artery occlusions: follow-up results in 103 patients." *Radiology* 194: 745 (1995)
Vorwerk,D. et al. "Venous stenosis and occlusion in hemodialysis shunts: follow-up results of stent placement in 65 patients." *Radiology* 195: 140 (1995)

## Claims

1. A graft comprising a tubular body with an inner and outer surface being open on both ends and defining a diameter with a perforated membrane fixed to its inner wall.
1 b. A graft according to claim 1 whose outer wall may be of a solid material, or a mesh made of any material.

2. A graft according to claim 1, wherein the inner membrane is at one or several places aligned to the inner wall of the graft, and/or may be circular around the inner circumference.

3. A graft according to claim 1, wherein the inner membrane has any other conceivable shape in the cross-section suited for releasing agents into the lumen of said graft.

4. A graft according to claim 3, wherein the inner membrane can be penetrated or passed with an agent by manipulation from outside of the passageway where said graft is placed and/or from inside or outside of the body in which said graft is installed.

5. A graft according to claim 4, wherein the inner membrane is accessible through a port, which itself is accessible by means of a access device, injection needle, or permanent connection.

6. A graft according to claim 5, wherein the port is accessible by punction of a member with a needle or other object or may also be accessible by means of any other mechanism which can provide access.

7. A graft according to claims 1 to 6, where the cuff, port, or ports and/or inner membrane are adorned with markers which may be chosen so that they are visible by various diagnostic methods.

8. A graft according to claim 7, wherein access to the port can be made temporarily or by permanent attachment to a subcutaneous connecting device.

9. A graft according to claims 1 to 8, which may be implanted into a blood vessel or any other body passageway or any other artificial body passageway or orifice by means of endoscopy, interventional radiology, or any other surgical manipulation.
